Europäisches Patentamt

European Patent Office

Office européen des brevets

(11) Publication number: **0 127 276**
**A2**

## EUROPEAN PATENT APPLICATION

(21) Application number: **84301909.2**

(22) Date of filing: **21.03.84**

(51) Int. Cl.³: **C 07 D 213/80**
**C 07 D 239/28, C 07 D 217/26**
**C 07 D 277/56**

(30) Priority: **31.03.83 GB 8309066**

(43) Date of publication of application:
**05.12.84 Bulletin 84/49**

(84) Designated Contracting States:
**DE FR GB IT**

(71) Applicant: **IMPERIAL CHEMICAL INDUSTRIES PLC**
**Imperial Chemical House Millbank**
**London SW1P 3JF(GB)**

(72) Inventor: **Head, Robert Arthur**
**68 Halton Road**
**Upton-by-Chester Chester CH2 1SN(GB)**

(72) Inventor: **Ibbotson, Arthur**
**29 Simmondley New Road**
**Glossop Derbyshire SK13 9LP(GB)**

(74) Representative: **Stephenson, Kenneth et al,**
**Imperial Chemical Industries PLC Legal Department:**
**Patents PO Box 6**
**Welwyn Garden City Herts, AL7 1HD(GB)**

(54) Carbonylation process.

(57) A method for the preparation of heterocyclic carboxylic acid esters by reacting a heterocyclic halide with carbon monoxide and an organic hydroxy compound in the presence of a carbonylation catalyst and an acid acceptor.

EP 0 127 276 A2

Croydon Printing Company Ltd

Qm. 32458/sp.
0127276

TITLE MODIFIED
see front page

## CHEMICAL PROCESS

This invention relates to a chemical process and more particularly to a method for the preparation of heterocyclic carboxylic acid esters.

According to the invention, there is provided a method for the preparation of a heterocyclic carboxylic acid ester which comprises reacting a heterocyclic halide with carbon monoxide and an organic hydroxy compound in the presence of a carbonylation catalyst and an acid acceptor.

The heterocyclic halides used in the method of the invention are heterocyclic compounds having one or more halogen atoms directly attached to carbon atoms in the heterocyclic ring. They are known materials and may be prepared using methods that have been fully described in the prior art. In particular, there may be mentioned heterocyclic halides containing at least one nitrogen atom in the heterocyclic ring which may optionally be fused or linked to one or more other carbocyclic or heterocyclic rings. Example of heterocyclic halides include halogen-substituted pyridines, quinolines, isoquinolines, thiazoles and pyrimidines which may be monohalogeno compounds such as chloro-, bromo-, and iodo- compounds or dihalogeno compounds such as dichloro-, dibromo-, di-iodo- and bromochloro- compounds.

In general, the reactivity of the halide increases with the atomic weight of the halogen atom but since cost increases in the same order, choice of starting material is often a matter for compromise, bromides generally being preferred. In addition to one or more halogen atoms, the heterocyclic ring may carry other substituents of an inert nature, for example optionally substituted alkyl, aryl or carbamoyl groups, any substituents thereon being inert under the carbonylation reaction conditions. Other inert substituents which may be present in the heterocyclic ring include carboxy, cyano, hydrocarbyloxycarbonyl, such as alkyloxycarbonyl, or trifluoromethyl groups. Particularly useful starting materials include 3,5-disubstituted pyridines, for example 3,5-dibromopyridines and 3-cyano- and 3-ethoxycarbonyl-5-bromopyridines.

Organic hydroxy compounds which may be used include alcohols and phenols. Suitable alcohols include aliphatic alcohols, for example those having from 1 to 20 carbon atoms, the aliphatic chain being either linear or branched where 3 or more carbon atoms are present. Other suitable alcohols include cycloaliphatic and arylaliphatic alcohols. As examples of suitable alcohols, there may be mentioned methanol, ethanol, isopropanol, octanols, isodecanol, hexadecanols, iso-octadecanol, cyclohexanol and benzyl alcohol. Suitable phenols include phenol itself and cresols. If desired, the organic hydroxy compound may be a polyhydroxy compound, for example ethylene glycol, glycerol, trimethylolpropane and polyoxyalkylene polyols.

The amounts of carbon monoxide and hydroxy compound employed should generally be at least one molar proportion of each per equivalent of reactive halogen atom but in many cases, for example, if the reaction zone is pressurised with carbon monoxide or if excess hydroxy compound is employed as solvent, the amounts will be much larger than this.

Carbonylation catalysts which may be used are well known in the art and include compounds of Group VIII metals. The preferred catalysts are palladium compounds which may be introduced into the reaction zone as oxides, salts, for example palladium (II) chloride or palladium (II) acetate or complexes, for example palladium acetylacetonate, potassium tetra-chloropalladate or nitrile complexes such as $PdCl_2 (RCN)_2$ wherein R is alkyl, for example methyl, or aryl, for example phenyl. It is possible that these compounds are converted at least partially to carbonyl complexes under the conditions of reaction. The catalytic activity of these palladium compounds may be increased by also introducing into the reaction zone an agent capable of stabilizing the compound in solution such as an organophosphine, for example triphenyl-phosphine. Alternatively, the catalyst may be introduced as a palladium-organophosphine complex, for example $Pd(PR_3)_4$, $PdCO(PR_3)_3$, $Pd(OAc)_2(PR)_2$ and $PdX_2(PR_3)_2$ wherein R has the meaning given above and X is a halogen atom, for example chlorine or bromine. If desired, the catalyst may be heterogenised by attaching a palladium compound to an appropriate catalyst support such as a phosphonated vinyl polymer. The heterogeneous catalysts can be recovered and recycled.

The carbonylation catalyst is used in a catalytically effective amount, suitable amounts usually being in the range 0.01 to 10% molar based on the heterocyclic compound. In general, catalyst concentrations of from 0.1 to 1% molar, based on the heterocyclic compound, give, the best compromise taking account of reaction rate and catalyst cost.

Suitable acid acceptors are basic materials which are capable of reacting with the hydrogen halide by-product but which take no other part in the reaction. As examples of acid acceptors there may be mentioned organic tertiary bases, for example triethylamine and alkali and alkaline earth metal salts of weak acids, for example sodium carbonate, sodium bicarbonate and potassium acetate. It is particularly convenient to use an inexpensive alkali such as sodium carbonate in conjunction with a minor amount of an organic base such as triethylamine.

Although it is not essential, the reaction may be performed, if desired, in a solvent which may conveniently be an excess amount of one of the essential reaction mixture ingredients, for example the hydroxy compound or an organic tertiary base when used as acid acceptor. Any other solvents used must be inert towards the reaction mixture ingredients and reaction products. Examples of suitable inert solvents include hydrocarbons, for example aliphatic hydrocarbons, benzene and toluene and ethers.

The reaction may be performed at ambient or elevated temperatures, for example up to 200°C. A particularly useful temperature range is from 50 to 150°C and especially from 80 to 120°C. Pressure may be atmospheric or higher, for example up to 100 atmospheres with a preference for 1 to 20 atmospheres.

When the reaction is complete, the heterocyclic carboxylic acid ester may be isolated and, if necessary, purified using conventional techniques. Also, the catalyst may be recovered for re-use.

The esters may be used as chemical intermediates. Certain pyridine mono- and di- carboxylic acid esters are metal complexing agents. In particular, the use of certain esters of pyridine carboxylic acids in the solvent extraction of metals such as copper, cobalt, cadmium and zinc is described in European Patent Application, Publication No. 0 057 797. Accordingly, the invention is particularly valuable for the preparation of the esters of pyridine carboxylic acids described in European Patent Application, Publication No. 0 057 797.

The invention is illustrated but not limited by the following Examples.

Example 1

A mixture of 3,5-dibromopyridine (4 g, 17 mmol), triethylamine (7.5 ml, 54 mmol), $PdCl_2(PPh_3)_2$ (0.29 g, 0.4 mmol) and ethanol (40 ml) was made in a glass liner containing a magnetic follower. The glass liner and contents were transferred to a stainless steel autoclave where the mixture was stirred by means of a rotating magnet directly below the autoclave base. After sealing, the vessel was pressurised to 100 psi with carbon monoxide and heated to 100°C for 10 hr before being cooled to room temperature. On venting off excess gases, the glass liner was removed and found to contain a pale yellow solution and a large amount of colourless crystals identified as triethylamine hydrobromide. The contents of the glass liner were washed into a flask using ethanol and volatiles were removed using a thin flim evaporator. The crude pale yellow product was dissolved in methylene chloride (100 ml) which was washed with 4 x 150 ml of deionized water to remove triethylamine hydrobromide. At this stage, yellow crystals of palladium compound were deposited which could be filtered off and recycled without loss of activity. The methylene chloride solution was dried over anhydrous sodium sulphate before being stripped to dryness to give 3.9 g of off white crystals which were shown by ir, 'Hnmr and HPLC to be essentially pure ("95%) diethyl ester of dinicotinic acid.

The following Table gives further Examples of the reaction of a heterocyclic halide, in the amount shown, with carbon monoxide (100 psi) in ethanol (40 ml) containing $PdCl_2(PPh_3)_2$ (0.3g) and triethylamine (7.5 ml) at 100°C as described in Example 1. The product compositions were determined by infra-red and/or HPLC.

| EXAMPLE | HETEROCYLIC HALIDE | REACTION TIME (h) | PRODUCT COMPOSITION |
|---------|--------------------|-------------------|---------------------|
| 2 | 5-bromonico-tinonitrile(6g) | 8 | ethyl 5-cyanonico-tinate(100%) |
| 3 | ethyl 5-bromo-nicotinate(5g) | 3 | diethyl dinico-tinate(100%) |
| 4 | 4-bromo-isoquinoline(4.5g) | 6 | 4-ethoxycarbonyl-isoquinoline(100%) |
| 5 | 5-bromo-pyrimidine(5g) | 3 | 5-ethoxycarbonyl-pyrimidine(100%) |
| 6 | 5-bromo-thiazole(19g) | 8 | 5-ethoxycarbonyl-thiazole("95%) |

EXAMPLE 7

Using the procedure, catalyst and base described in Example 1, isodecyl 5-bromonicotinate (25g) was reacted with carbon monoxide in isodecanol for 12 hours giving a product containing at least 95% of di-isodecyl dinicotinate.

The following Table given further Examples of the reaction of 3,5-dibromopyridine (4.0g) with carbon monoxide (100 psi) in ethanol (50ml) containing the indicated catalysts and acid acceptors at 100°C.

| EXAMPLE | Pd SOURCE | REACTION TIME(h) | BASE |
|---------|-----------|------------------|------|
| 8 | $Pd\ Cl_2(PPh_3)_2$ | 10 | $Na_2CO_3(2.5g)$ |
| 9 | " | 3 | triethylamine(7.5ml) |
| 10 | $Pd(PPh_3)_4$ | 3 | " |
| 11 | $Pd\ Cl_2(PPh_3)_2$ | 3 | $Na_2CO_3(2.5g)+NEt_3(0.3ml$ |

In each case, 0.3g of palladium compound was used and in each case the dibromopyridine was completely converted to diethyl dinicotinate. In Example 8, the conversion to diethyl dinicotinate after 3 hours reaction was 30%.

EXAMPLE 12

Using the reaction conditions described in Example 1, 3,5-dibromopyridine (5g) was reacted with carbon monoxide in ethanol (40 ml) in the presence of triethylamine (7.5 ml) and a catalyst (1.2 g) containing 4% palladium. After 10 hours reaction, the product contained diethyl dinicotinate (21%), ethyl 5-bromonicotinate (39%) and 3,5-dibromopyridine (40%).

The catalyst was prepared as follows:

Triphenylphosphine bound to styrene-divinylbenzene copolymer beads (3g) was added to $Pd(Cl)_2(PhCN)_2$ (10g) dissolved in acetone (100ml) and the mixture heated under reflux for 6 hr. The beads were filtered off, washed several times in warm acetone and carefully dried under high vacumn to give a light grey catalyst (Pd=4.0%).

CLAIMS

1. A method for the preparation of a heterocyclic carboxylic acid ester which comprises reacting a heterocyclic halide with carbon monoxide and an organic hydroxy compound in the presence of a carbonylation catalyst and an acid acceptor.

2. A method according to claim 1 wherein the heterocyclic halide has one or two bromine atoms directly attached to carbon atoms in a nitrogen-containing heterocyclic ring.

3. A method according to claim 2 wherein the heterocyclic ring is a pyridine ring.

4. A method according to claim 2 wherein the halide is 3,5-dibromopyridine.

5. A method according to claim 1 wherein the organic hydroxy compound is an aliphatic alcohol having from 1 to 20 carbon atoms.

6. A method according to claim 1 wherein the carbonylation catalyst is a palladium-organophosphine complex.

7. A method according to claim 1 wherein the reaction is performed at a temperature in the range from 50 to 150°C.

8. A method according to claim 7 wherein the reaction is performed at a temperature in the range from 80 to 120°C.